# EUROPEAN PATENT SPECIFICATION

(11) **EP 2 817 016 B1**
(45) Date of publication and mention of the grant of the patent: **01.04.2020**
(21) Application number: 12868255.6
(22) Date of filing: 10.02.2012
(51) Int. Cl.: A61K 36/21, A23K 10/30, A23K 50/75

(54) **METHOD FOR IMPROVING QUALITY OF POULTRY MEAT**
VERFAHREN ZUM VERBESSERN DER HÜHNERFLEISCHQUALITÄT
MÉTHODE POUR AMÉLIORER LA QUALITÉ DE LA VIANDE DE POULET

(43) Date of publication of application: 31.12.2014
(73) Proprietor: Khirug, Stanislav, 01360 Vantaa (FI)
(72) Inventor: KHIRUG, Sergey, FI-01360 Vantaa (FI); VYSHTAKALIUK, Alexandra, Kazan 420088 (RU)
(74) Representative: Heinonen & Co
(86) International application number: PCT/FI2012/050130
(87) International publication number: WO 2013/117799

(56) References cited:
- EP-A1- 1 222 861
- WO-A1-03/013495
- WO-A1-2007/098582
- CN-A- 102 578 383
- JP-A- 2003 063 972
- RU-C1- 2 160 994
- RU-C2- 2 168 908
- US-A1- 2011 064 844
- TATIYA A U ET AL: "Phytochemical investigation and immunomodulator activity of Amaranthus spinosus Linn", INDIAN JOURNAL OF PHARMACEUTICAL EDUCATION AND RESEARCH, ASSOCIATION OF PHARMACEUTICAL TEACHERS OF INDIA IND, IN, vol. 41, no. 4, 1 January 2007 (2007-01-01), pages 337-341, XP009186120, ISSN: 0019-5464
- MASANOBU HIBI ET AL: "Amaranth Grain Inhibits Antigen-Specific IgE Production Through Augmentation of the IFN-[gamma] Response in vivo and in vitro", CYTOTECHNOLOGY, KLUWER ACADEMIC PUBLISHERS, DO, vol. 43, no. 1-3, 1 November 2003 (2003-11-01), pages 33-40, XP019236814, ISSN: 1573-0778, DOI: 10.1023/B:CYTO.0000039908.34387.D3
- DATABASE WPI Week 200149 Thomson Scientific, London, GB; AN 2001-456192 XP002744781, & RU 2 170 096 C1 (CHERNEKHOVSKAYA N E) 10 July 2001 (2001-07-10)
- LIN B F ET AL: "Amaranthus spinosus water extract directly stimulates proliferation of B lymphocytes in vitro", INTERNATIONAL IMMUNOPHARMACOLOGY, ELSEVIER, AMSTERDAM, NL, vol. 5, no. 4, 1 April 2005 (2005-04-01), pages 711-722, XP027686360, ISSN: 1567-5769 [retrieved on 2005-04-01]
- DATABASE BIOSIS [Online] BIOSCIENCES INFORMATION SERVICE, PHILADELPHIA, PA, US; 2006, GONOR K V ET AL: "The influence of a diet with including amaranth oil on antioxidant and immune status in patients with ischemic heart disease and hyperlipoproteidemia", XP002744782, Database accession no. PREV200700180094 & GONOR K V ET AL: "The influence of a diet with including amaranth oil on antioxidant and immune status in patients with ischemic heart disease and hyperlipoproteidemia", VOPROSY PITANIYA, vol. 75, no. 6, 2006, pages 30-33, ISSN: 0042-8833
- PISARIKOVA B ET AL: "The use of amaranth (genus Amaranthus L.) in the diets for broiler chickens.", VETERINARNI MEDICINA, vol. 51, no. 7, 2006, pages 399-407, XP002754364,
- RANGARAJAN, A. ET AL.: 'Iron bioavailability from Amaranthus species: 2. Evaluation using haemoglobin repletion in anaemic rats.' JOURNAL OF THE SCIENCE OF FOOD AND AGRICULTURE vol. 78, no. 2, October 1998, pages 274 - 280, XP003031287
- DUTTA, A. ET AL.: 'Comparative analysis of aqueous extracts of amaranth and coriander in scavenging free radical activity and protection of DNA against oxidative damage.' CHIANG MAI JOURNAL OF SCIENCE vol. 38, no. 4, October 2011, pages 560 - 571, XP055161362
- JAYAPRAKASAM, B. ET AL.: 'Tumor cell proliferation and cyclooxygenase enzyme inhibitory compounds in Amaranthus tricolor.' JOURNAL OF AGRICULTURAL AND FOOD CHEMISTRY vol. 52, no. 23, November 2004, pages 6939 - 6943, XP002427570
- GIRIJA K. ET AL.: 'Anti-diabetic and anti-cholesterolemic activity of methanol extracts of three species of Amaranthus.' ASIAN PACIFIC JOURNAL OF TROPICAL BIOMEDICINE vol. 1, no. 2, April 2011, pages 133 - 138, XP055080099
- MAIYO, Z. C. ET AL.: 'Phytochemical constituents and antimicrobial activity of leaf extracts of three Amaranthus plant species.' AFRICAN JOURNAL OF BIOTECHNOLOGY vol. 9, no. 21, 24 May 2010, pages 3178 - 3182, XP055080101
- FASUYI, A. O. ET AL.: 'Protein supplementary quality of vegetable leaf meal (Amaranthus cruentus) in the diets of laying hens: egg laying performance, egg quality and haematological implications.' JOURNAL OF FOOD, AGRICULTURE & ENVIRONMENT vol. 5, no. 3 & 4, July 2007, pages 294 - 300, XP055166146
- POND, W. G. ET AL.: 'Nutritive value of a vegetable amaranth cultivar for growing lambs.' JOURNAL OF ANIMAL SCIENCE vol. 67, no. 11, November 1989, pages 3036 - 3039, XP055080107

## Description

### FIELD OF THE INVENTION

The present invention relates generally to methods of administering a nutraceutical composition comprising as an active ingredient a substance, extracted from phytomass of genus *Amaranthus* L., as a feed supplement for broiler chickens, and methods of application of said feed supplement in poultry, in particular, to improve quality of poultry meat.

### BACKGROUND

A professional term 'nutraceutical', that has been evolved recently, combines the term 'nutrient' and the term 'pharmaceuticals', and is used herein to denote utility in nutritional, pharmaceutical and veterinary fields of application.

Since the complete ban on antibiotic feed additives for animal and poultry feeding in agriculture within the European Union in 2006, the interest in developing of phytogenic, i.e. of plant origin, feed additives continues to grow. It is of general knowledge that plants, especially that of medicinal or aromatic origin, contain biologically active substances, which modes of actions on the organism of living species is yet not fully studied. However, phytogenic food supplements are known to possess manifold effect on the organism, contributing to the improvement of nutritive performance of total diet and at the same time acting as bioregulators of main functional systems of the organism.

Plants of genus *Amaranthus* L generally known as amaranth are widely spread and well known since Aztecs. Amaranth grains have been researched during last decades for particularly high squalene content in their lipid fraction. It is also known, that amaranth phytomass is rich on flavonoids, represented particularly by rutin, which is, from therapeutical point of view is powerful antioxidant and from the pharmacological point of view is important for increasing the strength of blood capillaries and regulating their permeability. *Amaranthus paniculatus* and *A. cruentus* were shown to be a particular good source for flavonoids. Studies on Amaranth extracts as a diuretic agent were undertaken (Martirosyan et al, Pharmacological Properties of Amaranthus, Legacy 15:2003 (1)). Same research group had worked out the use of Amaranth in modern diet for humans and developed a technology of extracting oil from amaranth seeds by hexane.

Antimicrobial agent and composition containing extracts of plants of genus *Amaranthus*, namely *A. caudatus*, *A. cruentus*, for use as a food, medicine or cosmetic additive, is disclosed in JP11255612A.

US Patent 5186963 discloses a dietary composition for infants and adults containing processed seeds of plants of family *Amarantaceae* as a protein source, along with fats, carbohydrates and a group of vitamins. The amount of amaranth flour in said composition was 30g per 100 ml.

The use of amaranth plant as a feed additive for farm animals is known from patent RU2374898. For the preparation of said additive amaranth oilcake has been used. The method of feeding of young chicken employing administering a vitamin additive derived from amaranth phytomass is disclosed in RU2160994. It was also demonstrated, that Amaranth vitamin-grass meal has stimulative effect on reproductive system development in replacement pullets and increases laying ability in laying hens in initial period of oviposition (Vyshtakalyuk et al., Agrobiology 2 (2010), 45-51).

Publication by Pisarikova et al (Veterinarni Medicina 51 (2006), No. 7, 399-407) concerns use of amaranth herbs as a feed supplement for poultry and farm animals. According to the teaching of this publication, diets with amaranth have no effect on percentage yield and on the quality of carcass or selected indicators of chemical composition of meat.

A number of publications discussed further below provide a general technical knowledge on other, than amaranth-based, feed additives for improving poultry and cattle meat quality. None of these publications discloses use of amaranth plant for improving poultry meat.

Thus, WO 2007/098582 relates to a feed/food supplement that enhances quality of poultry meat (in terms of weight gains and reduced stress during production/transport, as well as improved flavour, taste, tenderness in various cuts of poultry meat). Feed supplement of disclosed herein comprises omega 2 or omega 6 rich fatty acids derived plant oil or plant seed (flax or canola).

WO 2003/013495 discloses stress-reducing formulations in meat-producing animals. Disclosed formulations contain several natural ingredients, such as valerian root, for example.

EP 1 222 861 discloses a feed supplement that reduces pre-slaughter stress effects, such as catching, fastening and transporting, in animals and (poultry) birds.

US 2011/0064844 provides for a poultry diet that can soften the flesh quality of poultry meat and improve the texture.

An improvement of the efficiency and cost-effectiveness of farm animal and poultry production by means of feeding said livestock still remains to be an economic priority. It would be therefore highly advantageous to provide a product with a manifold function, which may act as a health-safe and cost-effective feed supplement. In addition, it would be desirable, that above mentioned product would be obtained from easily available raw material and by means of a process that is economical to operate.

### SUMMARY OF THE INVENTION

The present invention relates to methods of application in poultry of a nutraceutical composition comprising a substance, extracted from fresh, ensiled or dried phytomass of genus *Amaranthus* L as an active ingredient.

In particular, the invention concerns a method for improving quality of poultry meat, in accordance to what is defined in the independent claim 1.

In an embodiment of the invention a nutraceutical composition, comprising as an active ingredient a substance, obtained from fresh, ensiled or dried phytomass of genus *Amaranthus*, in particular *A. cruentus*, by means of extraction is provided, wherein the extraction methods comprise an extraction by an aqueous solution of ethanol. The term "nutraceutical" herein is used in order to describe the broad and manifold influence of said composition to the organism of poultry, whereupon said composition may be applied as a nutritional supplement for poultry feed when administered in effective amount. The prerequisites for each particular method of application will be disclosed further.

In some examples, a phytogenic feed supplement is provided in the form of the above mentioned nutraceutical composition, said phytogenic feed supplement is suitable for administration for poultry in order to compensate possible deficiency of nutritional and/or vitamin components in feed of relatively healthy object. By administration of said phytogenic feed supplement to poultry, in particular, to broiler chickens, improved quality of poultry meat may be achieved.

In an embodiment of the invention a method, related to the improvement meat quality in poultry, is provided, said method comprises administration of the above mentioned phytogenic feed supplement in accordance with an individual dosage regime, poultry breed and/or age and life cycle phase of poultry stock.

The term "extract" refers in this disclosure to a liquid product, comprising phytomass-derived complex of extractive substances dissolved in an extracting agent.

Terms "flock" and "stock" applied to poultry in this disclosure are interchangeable and both refer to experimental and/or control poultry batches.

### BRIEF DESCRIPTION OF THE DRAWINGS

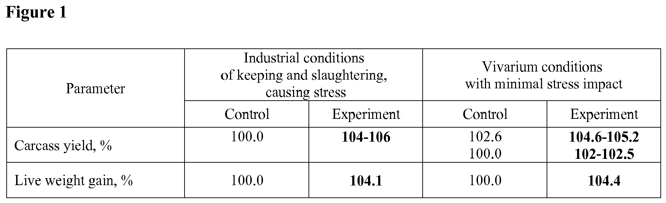

Figure 1 discloses table 1 as a comparative evaluation of anti-stress and metabolic effects caused by the nutraceutical composition used in the method of the invention in broiler chickens, raised in industrial- and in vivarium conditions (both experimental), in % from the corresponding control batch.

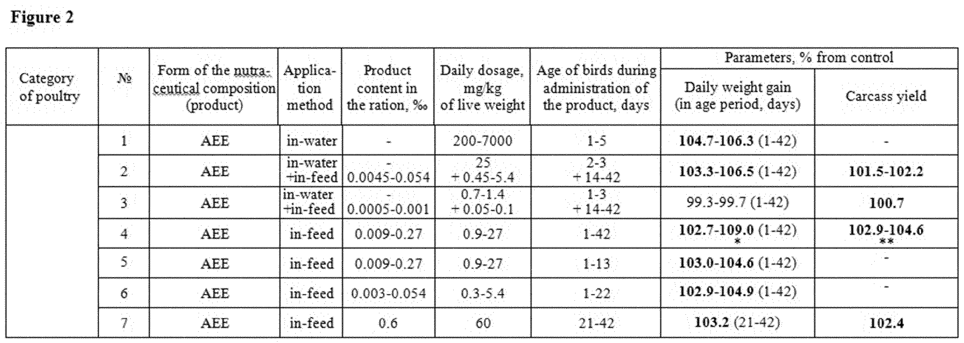

Figure 2 discloses table 2 as effect of the nutraceutical composition used in the method of the invention on growth intensity of meat chickens, as well as on slaughter meat yield, in % from the corresponding parameters calculated for poultry on a control diet, without addition of said composition.

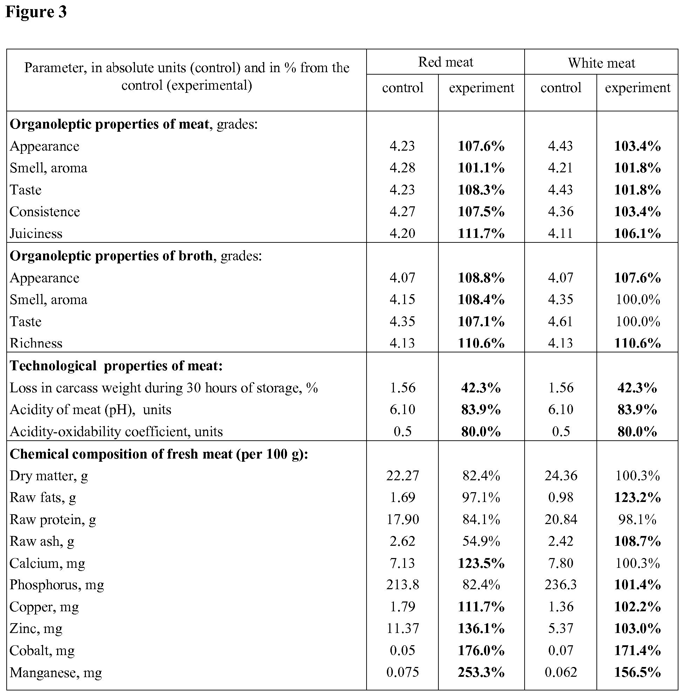

Figure 3 discloses table 3 as effect of the nutraceutical composition on quality of poultry meat, in % from the control ration, said composition was not administered thereto. Organoleptic properties of boiled meat and broth were evaluated in grades by five-grade scale; average value for four control and four experimental batches are outlined herein. Technological properties and chemical composition of meat are defined by the results of a single experiment.

### DETAILED DESCRIPTION OF THE INVENTION

In accordance with the embodiment of the invention the nutraceutical composition, comprising as an active ingredient a substance, obtained from phytomass of genus *Amaranthus* plants, is provided. For clarity purposes, further the plant of genus *Amaranthus* will be denoted as "amaranth". Said substance comprises biologically active compounds of amaranth and is obtained from amaranth phytomass by means of extraction. Fresh, dried or ensiled amaranth phytomass may be utilized as a raw material for the extraction process. Both conventional and modern techniques may be utilized to implement an extraction process, such as rotary-pulsation-, pressurized-liquid extraction, sub- and supercritical-fluid extraction, microwave-assisted-, ultrasonic- extraction techniques and the like.

A nutraceutical composition used in the method of the invention may comprise an active substance, obtained from amaranth phytomass as a supernatant fraction of the extraction process, herein, an extract. Said nutraceutical composition may thus be utilized in liquid-, substantially semisolid, e.g. as a suspension, and in substantially solid, e.g. dried, forms. The nutraceutical composition is provided in the form of an aqueous ethanol extract (AEE). The nutraceutical composition is hereby obtained as a result of an extraction by aqueous solutions of ethanol.

This formulation is expressed by the term 'nutraceutical composition' in further examples.

For those skilled in the art it is clear that a composition, comprising a substance, obtained from natural, i.e. plant source and containing biologically active compounds may not be strictly considered as a drug. However, it is also clear, that said biologically active compounds may naturally have a regulatory effect on the functional systems of an organism, thus possessing possible curative and protective properties.

The composition obtained from *Amaranthus* phytomass, as disclosed hereby, may thus be utilized as a curative and protective food supplement, which purpose is to supply nutritional components otherwise lacking from the feed ration, but it may be also utilized as means for the prevention and treatment of certain physiological conditions caused by dysfunctions of the organism. Dual nature of said composition is thus expressed by the term "nutraceutical".

The term "phytogenic feed supplement" thus refers to a feed supplement of a plant origin, obtained from amaranth in this disclosure.

The nutraceutical composition used in the method of the invention was studied in farm poultry, wherein term "farm poultry" denotes at least one of the following strains of broiler chickens: Hubbard Flex, Hubbard F15, Hubbard JV. Above mentioned farm poultry had been kept in cages. The nutraceutical composition, however, is suitable for administration to any other category of farm poultry.

For the purposes of the invention, the nutraceutical composition in the form of AEE prediluted in water may be introduced to feed formula (in-feed) of farm poultry in daily doses 0.05-60 mg of dry matter per kg of live weight. AEE may be admixed to drinking water and administered in-water in an amount equivalent to that, introduced to feed formula, or, alternatively, administered during an initial growth period for 3-5 days in daily doses 0.7-25 mg/kg, followed by introducing AEE to feed formula in daily doses 0.05-5.4 mg/kg. In the initial growth period and for infectious diseases preventive care, AEE as a drinking solution may be administered for 3-5 days in daily doses 200-7000 mg/kg.

All experiments concerning farm poultry were conducted in the conditions of industrial facilities, wherein poultry birds experience constant stress, related to the intensive bird-keeping technology. Keeping conditions for control and experimental poultry batches differed only by presence or absence of the nutraceutical composition of the invention in the ration. The product efficiency was evaluated on the background of real functional condition of poultry, which more or less differed from the physiological standards. Whether the condition parameters of some functional systems of the poultry organism were in physiological standards limits, the optimized performance of a functional system was considered as a measure for prophylactic action of the nutraceutical composition of the invention.

The term "broiler" refers in this disclosure to meat poultry breeds.

For the broiler chickens, in order to increase mass gain and to reduce negative stress-induced effects, the nutraceutical composition of the invention is administered in accordance with the following plan:
- either during the whole rearing period, starting from the 1^{st} day of life till slaughtering (39-45 days), or during certain rearing stages AEE is introduced into the feed formula (in-feed) in daily doses 0.003-0.27‰ (0.3-27 mg/kg). Instead of in-feed administration extracts may be admixed to drinking water in equivalent to that of in-feed amounts;
- in a time frame from the 1^{st} to 3-9^{th} days of life AEE is introduced to drinking water (in-water) in daily doses 200-7000 mg/kg;
- in a time frame from the 1^{st} to 3^{d} days of life AEE is introduced in-water in doses 0.7-25 mg/kg, followed by, starting from 14^{th} day of life till slaughtering, in-feed AEE introduction in doses 0.0005-0.054‰ (0.05-5.4 mg/kg);
- in a time frame from the 21^{st} to 45^{th} days of life AEE is introduced daily into feed formula in an amount of 0.6 ‰ (60 mg/kg).

The invention will be further described by reference to the following detailed examples.

For the clarity purposes, the content of said nutraceutical composition in the feed formula is expressed in terms of promille (‰), or one part per thousand, calculated per amount of dry matter contained in hydrolysate or extract; and the daily dose of said composition, expressed in milligrams per kilogram of body mass, refers to dry content of extractive substances in the extract, either upon administering as a drinking solution or upon feeding.

The data tables in all accompanying figures contain asterisks (from 1 to 3), that denote three different significance levels of probability (p) value for statistical hypothesis testing: 0.05, 0.01 and 0.001, correspondingly. In all data tables, unless otherwise stated, corresponding parameters for the control batch are taken as 100%; those parameters that differed from norm are underlined, and parameters, getting improved upon the effect of the nutraceutical composition of the invention are shown in bold.

### Example 1. Effects of the nutraceutical composition onto amount and quality of poultry meat in conditions of stress factors of varying intensity.

Anti-stress effect of the nutraceutical composition was studied on broiler chickens, in accordance to that disclosed previously in this document.

The nutraceutical composition in effective amounts was administered to broiler chickens, starting from 21^{st} day of life until slaughtering (39-44 days). The most of broiler stock was kept in standard industrial conditions, wherein birds usually experience stress first during capture and then during transportation at the day of slaughtering. One experiment was carried out in conditions of vivarium, 21 days old broiler chickens were relocated whereto from the poultry-house. Anti-stress effect of the nutraceutical composition in this experiment was evaluated by changes in chicken's live weight during first few days after relocation to vivarium. Anti-stress effect of said composition at the end of the rearing period in broilers was determined by difference in slaughter weight of chickens, raised in industrial conditions and experiencing stress before the slaughtering, compared to that of chickens, raised and slaughtered in vivarium in conditions of minimum stress. Carcass yield was determined by calculating the mass ratio of eviscerated carcass to live weight prior to stress impact before slaughtering (table 1, fig. 1).

The experiment on three weeks old broilers, conducted after the relocation-caused stress, demonstrated, that during first three days after the relocation event 22.2% of chickens lost body weight, and all others possessed a decreased weight gain dynamics. If in same conditions the nutraceutical composition would be included into chicken ration right after the relocation event, chickens would continue to gain weight without disturbance of weight gain dynamics.

It was shown that a carcass yield for broiler chickens of control batch that had experienced a minimal stress impact during slaughtering (in vivarium conditions) is 2.6% higher comparing to that of a control batch, slaughtered in industrial conditions (table 1, fig. 1). A carcass yield for chickens that have received the nutraceutical composition was 4-6% higher in industrial conditions, but only 2-2.5% higher in vivarium conditions in comparison to that for the corresponding control batches (table 1, fig. 1). Thereby, a positive effect of said nutraceutical composition on carcass yield is proportional to an intensity of an experienced stress impact that is indicative of an anti-stress nature of this effect. However, also in conditions of minimal stress impact, carcass yield for chickens, having said nutraceutical composition in their ration, was higher than that for control that may be indicative of other, than anti-stress, mechanisms, provided by this effect.

Results thus obtained indicate, that an increase in carcass yield as affected by said nutraceutical composition may be explained in terms of two mechanisms: an anti-stress impact of the nutraceutical composition that prevents a body mass loss during capture and transportation of poultry birds, and a metabolic effect, causing an increase of live weight in birds by building up the muscle and bone tissue. In vivarium conditions, wherein a pre-slaughtering stress impact on weight loss is brought to minimum, an increase in carcass yield, as affected by the nutraceutical composition of the invention, is mostly due to the metabolic effect. In industrial conditions, however, an increase in carcass yield, as affected by said nutraceutical composition, is due to both metabolic and anti-stress effects.

An existence of stress factor independent metabolic mechanism of action of said nutraceutical composition is confirmed by the fact, that the influence of said composition on live weight gain in poultry is practically the same in industrial- and vivarium keeping conditions, in contrast to its influence on carcass yield (table 1, fig. 1). In addition, said nutraceutical composition positively affects organoleptic and technological qualities of meat, causing improvement thereof. Thus, the fat content in meat increased by 17.6-22.3%, meat pH decreased by 16%, and acidity-oxidability coefficient decreased by 20% in comparison to that in control. These results are indicative of the improvement of functional state in chickens of experimental batches at the moment of slaughtering, of higher activity possessed by muscle enzymes, and of higher content of energetic substrates in muscle tissue. These differences between experimental and control chicken batches indicate, that adaptive potential of poultry organism gets improved under the influence of said nutraceutical composition, which makes poultry birds more prone to stress factors during capture, transportation and slaughtering.

The results obtained therefore are indicative of that said nutraceutical composition possesses an anti-stress effect, thus preventing live weight loss in conditions of stress impact during capture, transportation and slaughtering of poultry birds, which results in improved meat quality. Similarly said composition provides a protection from multiple stress factors of internal and external environment, such as nutritional, temperature or light factors, which inevitably affect poultry in conditions of intensive industrial farming. The possible mechanism of anti-stress action of said composition lies in an improvement of the adaptive potential of nervous and endocrine systems thus promoting a production of adaptive energy in an organism.

### Example 2. Method for improving growth performance, carcass yield and meat quality in broiler chickens.

In accordance to that disclosed previously in this document, the nutraceutical composition of the invention was administered to poultry feed formula in effective amounts.

An intensity of weight gain in broiler chickens was estimated by results of control weightings, by determination of an average weight of chickens at the moment of weighting and of a weight gain over a time period between weightings. Carcass yield for broilers was calculated as a mass ratio of eviscerated carcass to live weight of chicken before slaughtering. Product quality was evaluated by biochemical studies and by tasting of boiled meat and broth by the group of experts. Results are shown in tables 2 and 3 of Figures 2 and 3, respectively.

Administration of said composition to broiler chickens in effective amounts during the whole rearing period promotes an increase in live weight gain by 2.7-9.0% (table 2, fig.2, exp. 4). For achieving similar results with short term administration of said composition, the dosage thereof must be increased essentially, either in the beginning of the rearing period via addition into drinking water (table 2, fig. 2, exp. 1) or at the end of this period via addition into feed (table 2, fig. 2, exp. 7).

The nutraceutical composition also provides an increase in carcass yield for broilers by 2.9-4.6% in comparison to control (table 2, fig. 2, exp. 4).

A minimum effective dose for the extractive substance in aqueous ethanol extract in feed formula comprises only 0.003‰ (3 g per ton). Using the aqueous ethanol extract in lower doses is not effective (table 2, fig. 2, exp. 3).

Along with increasing broiler's productivity, administration of the nutraceutical composition may lead to quality improvement of products obtained, expressed in improved organoleptic, biochemical and technological parameters of meat (table 3, fig.3).

In experimental batches the nutraceutical composition was administered thereto almost all tasting evaluation grades for meat and broth organoleptic parameters have improved. Loss in carcass weight throughout 30 hours of refrigerated storage, specified by technological regulations of meat ripening, decreased by 41-48%, which is indicative of an improvement of technological properties of meat, in particular, of an increase of its moisture-keeping ability. Acidity of meat (pH) decreased by 2-16% and acidity-oxidability coefficient decreased by 20%, that is indicative of more effective meat ripening process upon its refrigerated storage, specified by the technology and intended to improve the product's flavor (table 3, fig.3).

In white meat an increased content of fats, ash, calcium and phosphorus were recorded, and in red meat an increased moisture load was recorded, that is indicative of an improved moisture-keeping ability and higher juiciness. In both white and red meat, the content of microelements, such as copper, zinc, cobalt and manganese, increases (table 3, fig.3). These results are indicative of an improvement of biological value of meat, obtained from poultry raised with the utilization of said nutraceutical composition.

The aforementioned examples provide a representative basis for teaching one skilled in art to utilize the present invention.

## Claims

1. A method for improving quality of poultry meat, comprising administration to poultry birds of a phytogenic feed supplement provided in the form of an aqueous ethanol extract and comprising, as a functional ingredient, a substance extracted from dry, fresh or ensilaged phytomass of *Amaranthus L.,* in particular of *Amaranthus cruentus L*.,
wherein said phytogenic feed supplement is administered, via drinking water, to newly hatched broiler chickens during the first days of life in an effective daily amount comprising 0,2-6 g per kg of live weight;
or
wherein said phytogenic feed supplement is administered, via feed formula, to broiler chickens during rearing period, in particular, from the first day of life and/or later until slaughtering in an effective daily amount comprising 0,3-60 mg, preferably 0,3-5,4 mg, per kg of live weight,
wherein the effective daily amount corresponds to an amount of dry substance extracted from phytomass of *Amaranthus.*

## Patentansprüche

1. Verfahren zur Verbesserung der Qualität von Geflügelfleisch, das die Verabreichung eines phytogenen Futterzusatzes an Geflügelvögel umfasst, der in Form eines wässrigen Ethanolextrakts bereitgestellt wird und als funktionellen Bestandteil eine Substanz umfasst, die aus trockener, frischer oder silierter Phytomasse von *Amaranthus L.* extrahiert wird, insbesondere von *Amaranthus cruentus L.,*
wobei der phytogene Futterzusatz frisch geschlüpften Masthühnern in den ersten Lebenstagen über Trinkwasser in einer wirksamen Tagesmenge verabreicht wird, die 0,2-6 g pro kg Lebendgewicht umfasst;
oder
wobei der phytogene Futterzusatz Masthühnern während der Aufzuchtperiode, insbesondere vom ersten Lebenstag und/oder später bis zur Schlachtung, in einer wirksamen Tagesmenge von 0,3-60 mg, vorzugsweise 0,3-5,4 mg je kg Lebendgewicht, über die Futterformulierung verabreicht wird,
wobei die wirksame Tagesmenge einer Menge an Trockensubstanz entspricht, die aus Phytomasse von *Amaranthus* extrahiert wurde.

## Revendications

1. Procédé d'amélioration de la qualité de la viande de poulet, comprenant l'administration à des volailles d'un supplément alimentaire phytogénique fourni sous la forme d'un extrait d'éthanol aqueux et comprenant, en tant qu'ingrédient fonctionnel, une substance extraite de phytomasse sèche, fraîche ou ensilée d'*Amaranthus L.,* en particulier d'*Amaranthus cruentus L.,*
dans lequel ledit supplément alimentaire phytogénique est administré, via l'eau de boisson, à des poulets de chair nouvellement éclos durant leurs premiers jours de vie en une quantité quotidienne efficace de 0,2 à 6 g par kg de poids vif ;
ou
dans lequel ledit supplément alimentaire phytogénique est administré, via une formule alimentaire, à des poulets de chair pendant la période d'élevage, en particulier, du premier jour de vie et/ou ultérieurement jusqu'à l'abattage en une quantité quotidienne efficace de 0,3 à 60 mg, de préférence 0,3 à 5,4 mg, par kg de poids vif,
dans lequel la quantité quotidienne efficace correspond à une quantité de substance sèche extraite de la phytomasse d'*Amaranthus*.
